Europäisches Patentamt

European Patent Office

Office européen des brevets

(19)

(11) Publication number: **0 160 145**
**A2**

(12) **EUROPEAN PATENT APPLICATION**

(21) Application number: **84303223.6**

(51) Int. Cl.⁴: **C 07 C 2/66**

(22) Date of filing: **11.05.84**

(30) Priority: **27.04.84 US 603034**

(43) Date of publication of application:
**06.11.85 Bulletin 85/45**

(84) Designated Contracting States:
**DE FR GB IT**

(71) Applicant: **Exxon Research and Engineering Company**
P.O.Box 390 180 Park Avenue
Florham Park New Jersey 07932(US)

(72) Inventor: **Bouncer, Heather Alexis**
155 North Front Street
Sarnia Ontario, N7T 7V5(CA)

(74) Representative: **Somers, Harold Arnold et al,**
ESSO Engineering (Europe) Ltd. Patents & Licences Apex
Tower High Street
New Malden Surrey KT3 4DJ(GB)

(54) Alkylation of aromatic molecules using wide pore, amorphous silica-alumina catalyst.

(57) Aromatic molecules such as benzene, toluene, ethylbenzene, naphthalene, tetralin etc. are selectively monoalkylated using heterogeneous wide pore, amorphous silica-alumina catalyst. High activity is maintained while achieving upwards of 89% monoalkylation of the aromatic starting material. The process has the advantage of not employing Friedel Craft catalysts, such as HF of $AlCl_3$ which are difficult to remove from the alkylated product stream and the presence of which could be detrimental to oil stability.

EP 0 160 145 A2

Croydon Printing Company Ltd.

## Description of the Invention

Wide pore, amorphous acidic silica-alumina has been found to be a highly selective, high activity catalyst for the monoalkylation of aromatic molecules in the liquid phase. Aromatic molecules such as benzene, toluene, o-, m-, p-xylene, ethylbenzene, tetralin, naphthalene, preferably ethylbenzene, and mixtures thereof are monoalkylated with linear olefins, both random and alpha-olefins, using a wide pore, amorphous acidic silica-alumina catalyst. The alkylation conditions include a temperature of about 25 to 250°C, preferably about 50 to 250°C, more preferably about 70 to 150°C, an olefin to aromatic ratio of 1:1 to 1:10, preferably 1:3 to 1:5, a pressure of at least about 150, preferably at least about 180 psig, preferably about 180 to 500 psig, more preferably about 180-250 at a WHSV of about 0.1 to 500 hr$^{-1}$, preferably 0.5 to 100 hr$^{-1}$.

The wide pore, amorphous acidic silica-alumina catalyst used in this alkylation process can be any amorphous acidic silica-alumina, wide pore material presently available on the market. For example, a commercial material available from Armak, Inc. as Ketjen HA 1.5E which when metal loaded is used as a hydrotreating catalyst has been found to be a very effective alkylation catalyst when not metal loaded.

In general, the wide pore, amorphous acidic silica-alumina catalyst may have a silica to alumina ratio of between 0.1:1 and 100:1, and an average pore diameter of about 40 Å or more. These materials are not zeolitic; that is, they are not crystalline with well defined structures. Useful catalysts may be in any commonly available physical form, for example, pellets, extrudates, etc.

## Description of The Figures

Figure 1 presents the catalyst activity profile plotting wt.% olefin converted as a function of time on stream at increasing temperature.

Figure 2 presents the wt.% olefin converted as a function of time, temperature and reaction pressure.

## Background

Conventional Friedel-Crafts alkylations of aromatic compounds with linear olefins, using $AlCl_3$, $BF_3$ or other Lewis acid as catalyst are known to produce alkylaromatic molecules in which virtually all, if not all, of the alkyl side chains are linear. That is, little or no structural isomerization of the linear olefin alkylating agent, or of the linear phenylalkane product, occurs under the influence of the alkylation catalyst. However, the use of such conventional catalysts in large processes is undesirable due to their corrosive nature, and the cost of continually regenerating or replacing the spent catalyst with fresh catalyst. It would be highly desirable to be able to effect the preparation of linear phenylalkanes containing little or no non-linear side product by using a heterogeneous non-corrosive catalyst.

In the past, when the alkylation of aromatic molecules with linear olefins has been carried out using heterogeneous silica alumina catalysts, these catalysts have been zeolites. That is, the catalysts have been crystalline materials with well defined chemical compositions and x-ray diffraction patterns.

The use of zeolites of various types as catalysts in alkylation reactions tends to produce a high proportion of the alkylaromatic isomer in which the aromatic moiety is attached to the second carbon of the alkyl side chain. This has been claimed to be highly desirable (U. S. Patent No. 4,301,316 and U. S. Patent No. 4,301,317). At the same time, however, a substantial proportion of the alkylaromatic material produced using zeolites as catalyst has non-linear side chains; that is, either the linear olefin and/or the alkylaromatic product has undergone skeletal isomerization under the influence of the zeolite catalyst. For example, in U. S. Patent No. 4,301,316 Mobil carries out the alkylation of benzene with n-$\alpha$-dodecene to obtain phenyldodecane products using various zeolite catalysts. These products generally contain a substantial amount of non-linear product; thus, using zeolite Beta, the phenyldodecane produced was only 53% linear (Example 2). Again, in U. S. Patent No. 4,301,317 Mobil alkylates benzene with n-$\alpha$-dodecene using H-ZSM-12 catalyst to obtain phenyldodecane containing 37% non-linear product (Example 7).

Non-linear alkylaromatics such as these are highly undesirable as lubricating oils or as detergent precursors since non-linear alkyl aromatic lubricating oils have low viscosity indices while alkylaromatic sulphonate detergents having non-linear alkyl side chains are non-biodegradable.

Commercial production of linear alkylbenzenes using Friedel-Crafts technology presently exceeds 500 million pounds per year. Alkylaromatic synthetic lubricating oils comprise only a small fraction of the lubricating oil market, but should increase in the future. Consequently, an efficient method for the preparation of high quality (that is, high viscosity index) alkylaromatics is required.

The Invention

It has now been discovered that the reaction of aromatic compounds with relatively long-chain alkylating agents, when carried out in the presence of certain large pore, non-crystalline amorphous acidic silica-alumina catalysts, will result in phenylalkanes of which only a small proportion has non-linear alkyl side chains. In addition, the major product of the reaction is that which results from mono-alkylation of the aromatic starting material; little poly-alkylation or olefin polymerization coproduction occurs.

The non-crystalline silica alumina catalysts utilizable in this process are characterized by channels or networks of pores, the radii of the openings to the channels ranging from about 20 Å to about 1,000 Å, and averaging from about 50 Å to about 500 Å.

The ratio of silica to alumina present in these amorphous catalysts is less important than the pore radii. The silica to alumina ratio can lie between about one and about 10, preferably between about 2 and 7. Two examples of catalysts which fall under the above description are the High Alumina (Si/Al = 3) and Low Alumina (Si/Al = 6) amorphous catalysts manufactured by Armak. The average pore radius in the High Alumina catalyst is about 100 Å, while that in the Low Alumina catalyst is about 188 Å.

The process is carried out by contacting the aromatic compound, which may be a substituted or unsubstituted aromatic, with the alkylating agent in the presence of the non-crystalline silica-alumina catalyst under suitable alkylation conditions. Preferred conditions include a temperature of between about 25°C and 500°C and a pressure of about at least 180 psig.

Suitable alkylating agents include linear alkyl halides, olefins and alcohols which contain at least 3 carbon atoms, preferably from about 4 to about 20 carbon atoms, more preferably about 10 to about 20 carbon atoms. The alkylating agents useful in the process of this invention include any aliphatic or aromatic organic compound which is capable of reacting with an aromatic compound. The preferred alkylating agents are olefins, most preferably linear olefins. Clearly, one can substitute any other hydrocarbon material which will generate unsaturated carbon atoms (e.g. olefins) in the presence of the disclosed alkylation catalysts.

The aromatic compounds which are to be alkylated with the foregoing alkylating agents to yield monoalkylated aromatics by the process disclosed herein are those which contain one to three aromatic rings, whether fused or not. These aromatic compounds may be unsubstituted, or they may already bear substituents on the ring structure. If substituted, the substituent may be an alkyl group having from one to ten carbon atoms therein, or may be a halide, an alkoxy, an aryl group, hydroxy, acid and so forth, or any combination of these or other substituents. The primary requirement is that there be at least one replaceable hydrogen atom on the aromatic ring. Consequently, the aromatics may be selected, for example, from benzene, toluene, o-, m-, p-xylene, ethylbenzene, n-, iso-propylbenzene, n-, iso-, tert-butylbenzene, tetralin, alkyl tetralin, naphthalene, etc. and mixtures thereof.

The catalysts used in the process of this invention may be a naturally occuring or synthetic amorphous, acidic silica-alumina material. The structures of the amorphous, acidic silica-aluminas suitable for use as catalysts in the process of this invention are such as to provide access to and egress from the interior of the catalyst particles by virtue of having channels or networks of pores, the openings thereto preferably having an average pore radius of from about $50^{\circ}$ to about 500 $\overset{\circ}{A}$, the actual pore radii ranging from about 20 $\overset{\circ}{A}$ to about 1,000 $\overset{\circ}{A}$. The ratio of silica to alumina in these catalysts can lie between about 1 and about 10, preferably between about 2 and about 7.

The catalyst useful in the conversion process of this invention have at least 10 percent of the cationic sites occupied by ions other than alkali or alkaline earth metals. Typical but non-limiting replacing ions include ammonium, hydrogen, rare earth, zinc, copper and aluminum. Of this group, particular preference is accorded ammonium, hydrogen, rare earth and combinations thereof. In a preferred embodiment, the catalysts are converted to the predominantly hydrogen form, generally by replacement of the alkali metal or other ion originally present with hydrogen ion precursors, e.g., ammonium ions, which upon calcination yield the hydrogen form.

The catalysts may be subjected to various chemical treatments, including alumina extraction and combination with one or more metal components, particularly the metals of Groups IIB, III, VI, VII and VIII. It is also contemplated that the amorphous catalysts may, in some instances, desirably be subjected to thermal treatment, including steaming or calcination in air, hydrogen or an inert gas, e.g., nitrogen or hydrogen. In a preferred embodiment, the catalyst in either the ammonium or hydrogen form is calcined in a dry atmosphere at a temperature between about $300°$ and $600°C$, most preferably between $400°$ and $500°C$, for 1 to 5 hours.

The process of this invention is conducted such that the organic reactants, i.e., the aromatic compound and the alkylating agent, are brought into contact with the catalyst in a suitable reaction zone, such as, for example, a flow reactor containing a fixed bed of the catalyst, under effective alkylation conditions. Such conditions include a temperature of between about $25°C$ and about $500°C$, a pressure of between about 150 psig and 5000 psig, and a feed weight hourly space velocity (WHSV g olefin:g catalyst 1 hr) of between about 0.1 and about 500. Preferred reaction conditions include a temperature within the approximate range $50°C$ to $250°C$, with a feed WHSV between 0.5 and 100. Although the reaction normally takes place at atmospheric pressure, the preferred pressure range extends from about 180 psig to about 500 psig, since it has been unexpectedly found that at pressures less than 180 psig, catalysts activity is decreased and catalyst deactivation can occur. The reactants are preferably in the liquid phase, and may be in either the vapour phase or the liquid phase and may be neat, i.e., free from intentional admixture or dilution with other materials, or may be brought into contact with the catalyst with the aid of carrier gases or diluents such as, for example, hydrogen or nitrogen.

The alkylation process described herein may be carried out as a batch type, semi-continuous or continuous operation utilizing a fixed bed or moving bed catalyst system. The preferred method of use is continuous operation.

The alkylation product may be purified, if necessary, and/or recovered from the starting materials or coproduced by products, if desired, by standard separation techniques. For example, the presence of unsaturated olefinic dimer in the alkylation product may be undesirable in applications where good oxidative stability is required. In this case, hydrogenation, either with an olefin-specific hydrogenation catalyst or with a hydrogenation catalyst which will saturate the olefin dimer and convert the alkylaromatics to naphthenes, may be desirable. Alternatively, purification may be carried out by physical separation techniques such as by distillation, or selective permeation through a perm-selective membrane, such as an asymmetric reverse osmosis polyimide membrane. Such a procedure for separating the alkylation product from the starting material and byproducts and the simultaneous separation of the alkylate product into its isomers using membranes is described and claimed in copending application Attorney Docket No. OP-2903, U. S. Serial No. 603,028, filed even date herewith.

The alkylaromatic produced is preferably the product of mono-alkylation of a light aromatic molecule. The relatively long chain will generally contain about 12-18 carbons, most preferably 14-16, carbons when the aromatic molecule which is alkylated is toluene or ethylbenzene. The alkylated aromatic material will usually contain a total of 21 to 28 carbon atoms.

The present alkylation process using the wide pore, amorphous acidic silica-alumina catalyst produces a product very rich in monoalkylated material, i.e., upwards of 89% monoalkylated material, with the balance being dialkylate and olefin dimer.

The long-chain mono-alkylated product is useful in the detergent industry. The use of certain monoalkylated alkyl benzenes, (i.e. dialkylbenzene materials containing one short (2-4 carbon) and one long (14-18 carbon) alkyl groups and containing a total of 23-28 carbons preferably 24-26 carbons) as specialty oil or lubricating oil basestocks or basestock additives is disclosed and claimed in copending application Attorney Docket No. OP-3016, U. S. Serial No. 603,032 , filed even date herewith. The dialkylbenzene products produced herein which are useful as synthetic specialty oil or lube oil basestock or basestock additive in accordance with OP-3016, U. S. Serial No. 603,032 can be used as produced; there is no need to separate the dialkylbenzene from the bulk reaction product mixture, other than to effect solvent recovery which can be handled by distillation and diolefin removal which can be handled by hydrogenation.

The following examples are provided to illustrate the process of this invention and are not to be interpreted as limitations on the scope of the present invention.

Example 1

A sample of amorphous, high alumina catalyst in its hydrogen form, 1.5 mm extrudates (Ketjen HA1.5E) was placed in a continuous reactor and calcined at 400°C for 2 hours in a stream of nitrogen gas. A feed stream containing toluene and $\alpha$-n-hexadecene (molar ratio 6.5/1) was passed over the catalyst at 145°C, 180 psig ($H_2$) pressure, $H_2$ flow rate 1.5 cu ft/hr and a WHSV of 1.0 $hr^{-1}$. Gas chromatographic analysis of the product stream after 88 hours continuous operation indicated conversion of $\alpha$-n-hexadecene was 93.9%. Selectivity to tolylhexadecanes was 65.54%, of which 1.95% was non-linear. Of the linear tolylhexadecanes produced, the amount of the 2-tolylhexadecane isomer product was 27.3%. These results are summarized in Table 1.

Example 2

The experiment described in Example 1 was continued for 258 hours, then the composition of toluene and $\alpha$-n-hexadecene in the feed stream was changed to a molar ratio of 5.2/1 (toluene/$\alpha$-n-hexadecene). All other parameters of the system remained as described in Example 1. After a total of 290 hours on stream, a sample of the product stream was subjected to gas chromatographic analysis. Conversion of α-n-hexadecene was 79.7%. Selectivity to tolylhexadecane was 76.6% of which 1.98% was non-linear. Of the linear tolylhexadecanes produced, the amount of the 2-tolylhexadecane isomer product was 36.0%. These results are summarized in Table 1.

Example 3

A sample of Ketjen HA1.5E catalyst was placed in a continuous reactor and calcined at 500°C for 2 hours in a stream if nitrogen gas. A feed stream containing toluene and α-n-hexadecene (molar ratio 6.5/1) was passed over the catalyst at 115°C, 180 psig ($H_2$) pressure, $H_2$ flow rate 1.5, and a WHSV of 1.0 $hr^{-1}$. Gas chromatographic analysis of the product stream after 62 hours continuous operation indicated conversion of α-n-hexadecene was 85.9%. Selectivity to tolylhexadecane was 84.2%, of which 0.82% was non-linear. Of the linear tolylhexadecanes produced, the amount of the 2-tolylhexadecane isomer was 40.4%. These results are summarized in Table 2.

Example 4

The experiment described in Example 3 was continued for 112 hours, then the composition of the feed stream was changed to a molar ratio of 4.9/1 (toluene/α-n-hexadecene). All other parameters of the system remained as described in Example 3. After a total of 130 hours on stream, a sample of the product stream was subjected to gas chromatographic analysis. Conversion of α-n-hexadecene was 76.2%. Selectivity to tolylhexadecane was 81.6% of which 0.81% was non-linear. Of the linear tolylhexadecanes produced, the amount of the 2-tolylhexadecane isomer product was 41.8%. These results are summarized in Table 2.

Example 5

The experiment described in Example 4 was continued for 452 catalyst hours, then the composition of the feed stream was changed to a molar ratio of toluene to $\alpha$-n-hexadecane of 3.0/1. All other parameters of the system remained as described in Example 3. After a total of 490 hours on stream, a sample of the product stream was subjected to gas chromatographic analysis. Conversion of $\alpha$-n-hexadecene was 54.8%. Selectivity to tolylhexadecanes was 73%, of which 0.42% was non-linear. Of the linear tolylhexadecanes produced, the amount of the 2-tolylhexadecane isomer product was 46.2%. These results are summarized in Table 2.

Example 6

The experiment described in Example 5 was continued to 542 catalyst hours, then bond-randomized n-hexadecene was substituted for $\alpha$-n-hexadecene, and the molar ratio of toluene to randomized n-hexadecene was 5/1. The reactor temperature was raised to 145°C, the WHSV remained at 1.0 $hr^{-1}$, the pressure remained at 180 psig, and the $H_2$ flow rate was 1.5 cu ft/hr. After a total of 662 hours on stream, a sample of the product indicated, by gas chromatographic analysis, that conversion of the olefin was 86.4%. Selectivity to tolylhexadecanes was 78.9%, of which 2.53% was non-linear. Of the linear tolylhexadecanes produced, the amount of the 2-tolylhexadecane isomer present was 25.6%. These results are summarized in Table 2.

## Table 1

ALKYLATION OF TOLUENE WITH $\alpha$-n-HEXADECENE. CATALYST CALCINED AT 400°C

| Example | Catalyst Hours | Reactor Temp., °C | Aromatic: Olefin Mole Ratio | Olefin | Olefin Conversion | Selectivity to Product of Mono-Alkylation | % Linear Product | Selectivity to 2-Tolyl Isomer |
|---------|----------------|-------------------|-----------------------------|--------|-------------------|-------------------------------------------|------------------|-------------------------------|
| 1 | 88 | 145 | 6.5/1 | $\alpha$-n-$C_{16}$= | 93.9% | 65.5% | 98.05 | 27.3 |
| 2 | 290 | 145 | 5.2/1 | $\alpha$-n-$C_{16}$= | 79.7% | 76.6% | 98.02 | 36.0 |

- 13 -

0160145

## Table 2

ALKYLATION OF TOLUENE WITH HEXADECENE.  CATALYST CALCINED AT 500°C

| Example | Catalyst Hours | Reactor Temp., °C | Aromatic: Olefin Mole Ratio | Olefin | Olefin Conversion | Selectivity to Product of Mono- Alkylation | % Linear Product | Selectivity to 2-Tolyl Isomer |
|---------|----------------|-------------------|------------------------------|--------|-------------------|---------------------------------------------|------------------|-------------------------------|
| 3 | 62 | 115 | 6.5/1 | $\alpha$-n-$C_{16}$= | 85.9% | 84.2% | 98.18 | 40.4 |
| 4 | 130 | 115 | 4.9/1 | $\alpha$-n-$C_{16}$= | 76.5% | 81.6% | 98.19 | 41.8 |
| 5 | 490 | 115 | 3.0/1 | $\alpha$-n-$C_{16}$= | 54.8% | 73.0% | 99.58 | 46.2 |
| 6 | 662 | 145 | 5.0/1 | Randomized -n-$C_{16}$= | 86.4% | 78.9% | 97.47 | 25.6 |

- 14 -

Example 7

100 ml (45.2 g) Ketjen HAl.5E catalyst (1/8" extrudates) was spread as thinly as possible on an 8" x 10" screen, and placed in an oven at 500°C for 2 hours which had a flow of breathing air of 7.0 cuft/hr. The hot catalyst was removed from the oven and stored in a nitrogen atmosphere.

The catalyst was placed in the unit, pressure tested cold at 200 psig with $N_2$ and $H_2$, then heated up to 200°C under a $N_2$ purge. The reactor was pressure tested at 200°C and 200 psig with $H_2$, then cooled to 60°C.

Alkylation of ethylbenzene with $\alpha$-n-hexadecene was carried out in a pilot plant unit operating upflow. Standard conditions were 180 psig ($H_2$), 1.5 cuft/hr $H_2$, with a feed consisting of a 5:1 molar ratio of ethylbenzene:$\alpha$-n-hexadecene. Temperature was the main variable, this being adjusted as required to obtain conversions of 80% or more. Initial temperature in the runs was 65°C. Temperature was then raised stepwise to the desired operating range of 105°C to 150°C. Previous studies have indicated that at temperatures above 150°C, undesirable olefin skeletal rearrangements occur. Generally, a WHSV of 1.0 $hr^{-1}$ (g. olefin: g. catalyst/hr) was used, but near the end of the run using Ketjen HAl.5E catalyst this was decreased to 0.8 $hr^{-1}$ to increase olefin conversion.

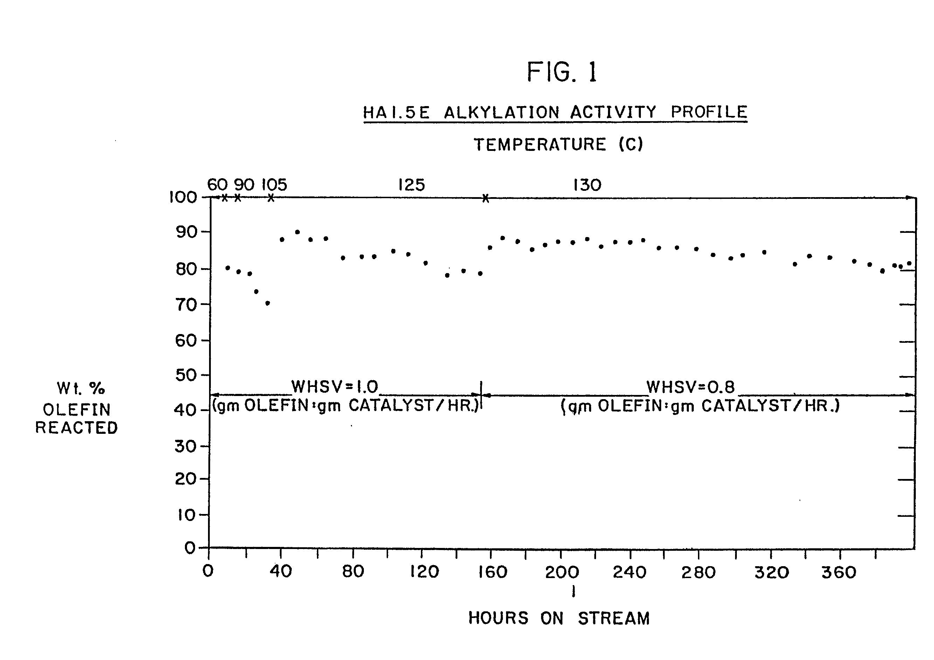

Figure 1 illustrates the percent $\alpha$-n-hexadecene conversion and the reactor temperature at various times during the alkylation run using Ketjen

HA1.5E catalyst. 80%+ olefin conversion was maintained at 125°C for 121 hours, and even at the end of this time (that is, at 154 hours) conditions were altered not because of catalyst deactivation, but simply to increase olefin conversion for product production.

The Ketjen alkylation product contains monoalkylation product (ethylhexadecylbenzene), a di-alkylation product (ethyldihexadecylbenzene) and hexadecene dimer, catalyst selectivities for these three products being typically 89 to 94%, 2.5 to 5.5% and 2.5 to 3.7%, respectively, as determined by gas chromatographic analysis (see Table 3).

Samples 238 to 246 from the run using the Ketjen catalyst were stripped to remove unreacted starting materials, then subjected to a modified D2440 oxidation test, to determine its oxidation stability. In this test, 50g of oil containing 0.06 wt% DBPC anti-oxidant and a copper wire oxidation catalyst is heated at 110°C while oxygen is passed through a water trap which retains the volatile acids which are produced as the oil degrades. The extent of oil degradation is determined by monitoring the amount of accumulated volatile acids in the water trap. The test is considered to be complete when the total volatile acid accumulated is equivalent to 5 mg KOH/g oil. The time required to reach this level of degradation was 168 hours, which reflects the presence of oxidatively unstable hexadecene dimer olefin in the sample. To ensure oxidative stability, this product would preferably be hydrogenated.

## Table 3

### PRODUCT DISTRIBUTION OBTAINED IN THE ALKYLATION REACTIONS

| Catalyst | Ketjen HA1.5E | | |
|---|---|---|---|
| Product | Mono-Alkylation Product | Di-Alkylation Product | Hexadecene Dimer |
| Sample Number | | | |
| Run 2-24 (hrs) | 92.77% | 3.57% | 3.66% |
| -54 (hrs) | 91.68% | 5.78% | 2.54% |
| -132 (hrs) | 94.36% | 2.50% | 3.14% |
| -246 (hrs) | 91.51% | 5.47% | 3.02% |
| -314 (hrs) | 89.70% | 4.99% | 3.17% |
| Run 2 - Samples 238-246 (hrs) inclusive | 91.78% | 5.17% | 3.05% |

## Table 4

### PHYSICAL PROPERTIES OF BULK ALKYLATION PRODUCTS (NOT HYDROGENATED)

| Catalyst | Ketjen HA1.5E |
|---|---|
| Sample Numbers | 238-246 |
| Viscosity, cSt, 40°C | 11.58 |
| 100°C | 2.99 |
| Viscosity Index | 112 |
| Pour Point, °C | -33 |
| Volatility (LV% off, 375°C) | 7 |

Example 8

The following example demonstrates the effect of pressure on the alkylation of a light aromatic molecule with a linear olefin, as well as the relative merits of using $H_2$ or $N_2$ as the gas.

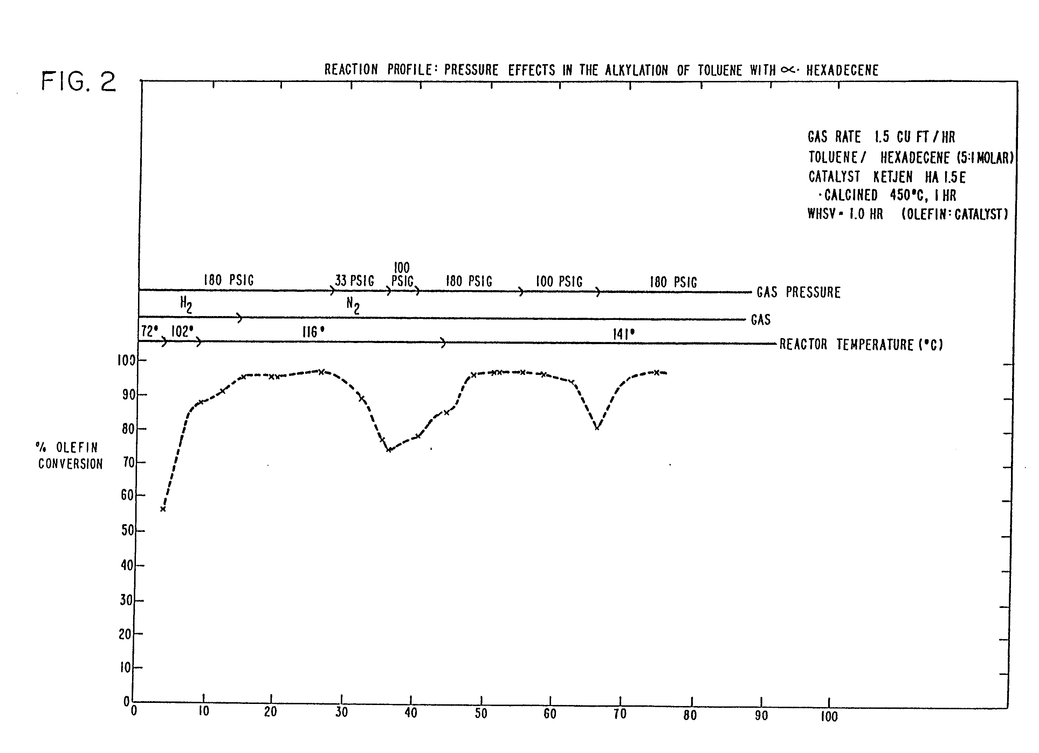

The feed consisted of toluene and $\alpha$-n-hexadecene (5:1 molar ratio). The catalyst was Ketjen HA 1.5E, calcined at 450°C for 1 hour. Throughout, the gas flow rate was 1.5 cu ft/hr, and the weight of olefin to weight of catalyst was 1.0 $hr^{-1}$. The conversion of olefin to alkylate was determined by gas chromatographic (GC) analysis of spot product samples. A plot of % olefin conversion with time on stream, including details of the gas used and its pressure, is presented in Figure 2.

At the beginning of the run, $H_2$ was the gas used, and the pressure was 180 psig. The temperature was raised from 72°C (1-4 hours on stream) to 102°C (4-9 hours on stream) to 116°C (9-15 hours) to obtain an olefin conversion of 95 weight percent. After 15 hours, the gas was changed from $H_2$ to $N_2$ (still at 180 psig) until a time of 28 hours on stream. This gas change, at the same pressure, did not alter the olefin conversion (this remained at about 95 weight percent from 15 to 28 hours on stream). At 28 hours the $N_2$ gas pressure was decreased to 33 psig, which immediately caused a drop in the olefin conversion to 74 weight percent at 36 hours on stream. This illustrates that $N_2$ gas pressure is important in maintaining olefin conversion. On raising the $N_2$ gas pressure to 100 psig (36-40 hours on stream) the olefin conversion increased slightly to 78 weight percent. On increasing the $N_2$ gas pressure to 180 psig (40-44 hours on stream) the olefin conversion increased to 83 weight percent. This illustrates that olefin conversion is dependent on $N_2$ gas pressure and that the use of low gas pressures leads to catalyst deactivation, since the original olefin conversion (95 weight percent) was not regained at the same reaction conditions.

To regain high olefin conversion, the reaction temperature was increased to 141°C (still at 180 psig $N_2$). Under these conditions, olefin conversion was 97 weight percent (44-55 hours on stream). After 50 hours on stream the $N_2$ pressure was decreased to 100 psig (55-66 hours on stream) and again olefin conversion decreased (to 81 weight percent). In this case, catalyst deactivation had not occurred since increasing the $N_2$ pressure again to 180 psig restored the olefin conversion to 97 weight percent.

Preferred reaction conditions therefore include a gas pressure of at least 180 psig to maintain olefin conversion and catalyst activity. An upper limit would be about 500 psig. Either $H_2$ or $N_2$ can be used as the gas. As $N_2$ appears to perform as well as $H_2$ (previous tests), $N_2$ would be preferred on the basis of cost and safety.

In this patent specification :

| | | |
|---|---|---|
| inch (or ") | = | 2.54 cm |
| pound | = | 0.45359 kg |
| cu. ft | = | 28.316 litre |
| Å | = | $1 \times 1^{-10}$m |
| psig | = | 6.895 kPa gauge |

D2440 oxidation test is the ASTM test D2440.

The co-pending patent applications herein referred to are as follows :

(1)     Co-pending application Attorney Docket No. OP-2903, U. S. Serial No. 603028 corresponds to our European patent application No.        filed on or about the same date as the present application and entitled: "Process for Separating Alkylaromatics from Aromatic Solvents and the Separation of the Alkylaromatic Isomers using Membranes" (inventors: L. E. Black and H. A. Boucher);

(2)     Co-pending application Attorney Docket No. OP-3016, U.S. Serial No. 603032 corresponds to our European Patent Application No.        filed on or about the same date as the present application and entitled : "Dialkylaromatics and Hydrogenated Dialkylaromatic Synthetic Lubricating and Speciality Oils" (inventor: H. A. Boucher)

CLAIMS :

1.    A process for the production of alkylated aromatic hydrocarbons comprising contacting an aromatic hydrocarbon with an alkylating agent, possessing carbon-to-carbon double bonds or capable of possessing carbon-to-carbon double bonds under the process conditions, in the presence of large pore non-crystalline amorphous acidic silica-alumina catalysts wherein said contacting is at a pressure of at least about 150 psig (i.e., at least about 1034.25 kPa gauge).

2.    The process of claim 1 wherein the reaction is conducted at a temperature of from about 25 to 500°C, a WHSV of from about 0.1 to 500 $hr^{-1}$ and an alkylating agent to aromatic molar ratio of from about 1:1 to 1:10.

3.    The process of claim 1 or claim 2 wherein the large pore non-crystalline amorphous acidic silica-alumina catalyst has a silica to alumina ratio of between about 0.1:1 and 100:1 and an average pore diameter of about 40 Å or more.

4.    The process of any one of claims 1 to 3 wherein the aromatic hydrocarbon is selected from benzene, toluene, o-, m-, p-xylene, ethylbenzene, n-, isopropyl benzene, n-, iso-, tert-, butylbenzene, tetralin, alkyltetralin, naphthalene and mixtures thereof.

5.      The process of any one of claims 1 to 4 wherein the alkylating agent comprises an olefin.

6.      The process of claim 5 wherein the alkylating agent comprises an olefin containing at least 3 carbons.

7.      The process of claim 6 wherein the alkylating agent comprises an olefin containing from 4 to 20 carbons.

# FIG. 1

## HA1.5E ALKYLATION ACTIVITY PROFILE

TEMPERATURE (C)

FIG. 2

REACTION PROFILE: PRESSURE EFFECTS IN THE ALKYLATION OF TOLUENE WITH ∝· HEXADECENE

GAS RATE 1.5 CU FT / HR
TOLUENE / HEXADECENE (5:1 MOLAR)
CATALYST KETJEN HA 1.5E
·CALCINED 450°C, 1 HR
WHSV = 1.0 HR (OLEFIN: CATALYST)

0160145